# EUROPEAN PATENT APPLICATION

(11) **EP 0 574 638 A1**
(43) Date of publication of application: **22.12.1993**
(21) Application number: 92500080.4
(22) Date of filing: 19.06.1992
(51) Int. Cl.: A61K 47/48, A61K 37/02

(54) **Soluble dextran-glutathione complexes, method of preparing same and their use**

(71) Applicant: EUROPHARMA, S.A., E-28042 Madrid (ES)
(72) Inventor: Sanchis Sánchez,Dolores, c/o EUROPHARMA, S.A., E-28042 Madrid (ES); Diaz Garcia-Maurino, Teresa, c/o EUROPHARMA, S.A., E-28042 Madrid (ES); Martin-Lomas, Manuel, c/o EUROPHARMA, S.A., E-28042 Madrid (ES)
(74) Representative: Elzaburu Marquez, Alberto

(57) **Abstract**

New soluble dextran-glutathione complexes and a method of preparing same. The complexes are characterized by a glutathione percentage of maximum 30% and a molecular weight in the range of 14.000 to about 40.000. The method is characterized in that it comprises subjecting a soluble dextran to oxidation, then to glutathione reaction to give the corresponding Schiff's base and, lastly, to chemical reduction.

The new soluble dextran-glutathione complexes are suitable for treating hepatopathies.

## Description

The present invention refers to new soluble dextran-glutathione complexes, to a method of preparing same as well as to their use in treating hetatopathies.

Glutathione (GSH-γ-L-glutamyl-L-cysteinyl-glycine) is widely distributed in nature and has been isolated from yeast, muscle and liver. GSH functions in the cell are to maintain normal redox potential, to scavenge peroxides, (which are toxic by-products of many metabolic reactions), and free radicals, and to detoxify drugs. GSH levels are diminished in cirrhotic patients and after the administration of ethanol to hmans, rats, and baboons. A depletion in liver GSH has been correlated with the development of liver fibrosis, cirrhosis, and tumor formation. Replenishment of cellular GSH levels by exogenous GSH administration is not feasible since this molecule cannot cross the cellular membrane. To overcome this problem, several permeable derivatives of GSH have been developed. (Meister A. and Anderson, M.E.: Glutatione; Annu. Rev. Biochem. 52; 711-760, 1983). Here we present the preparation of new GSH derivatives and their capacity to cros the plasma membrane. The latter is demonstrated by showing that the addition of these substances to isolated rat liver hepatocytes leads to a marked increase in cellular GSH even under conditions where the synthesis of GSH is inhibited by the administration of buthionine sulfoximine (BSO). These products have therefore a potential interest to be used as therapeutical agents to prevent the development of liver fibrosis, in the treatment of hepatopathies and to neutralize the toxic effect of a variety of drugs.

The preparation of the active substances now reported have been based on previous findings on the increase of half-lives of circulatory proteins by conjugation to soluble polymers (J.R. Mitchell, D.J. Jollow, W.Z. Potter, D.C. Davies, J.R. Gillette and B.B. Brodie, J. Pharmacol. Exp. Ther., 1973, 187, 185; B.H. Lauterburg and J.R. Mitchell, Hepatology, 1982, 2, 8; J. de Vries, Biochem. Pharmacol. 1981, 30, 399; G.M. Rosen, W.V. Singletary, E.J. Rauckman and P.G. Killenberg, Biochem. Pharmacol, 1983, 32, 2053). A previous study has shown that a dextran conjugate of GSH could be used as an intrahepatic delivery system for GSH (Y. Kaneo, Y. Fujihara, T. Tanaka, Y. Kozawa, H. Mori and S. Iguchi, Chem. Pharm. Bull. 1989, 37, 218). In this study GSH was covalently attached to dextran using a cyanogen bromide activation method (R. Axen and S. Ernback, Eur. J. Biochem 1971, 18. 351). We now have avoided this highly toxic activating agent by using modified dextrans in an adaptation of the method previously developed for coupling proteins to cellulose (C. Flemming, A. Gabert, and P. Roth, Acta Biol. Med.Ger. 1973, 30, 177).

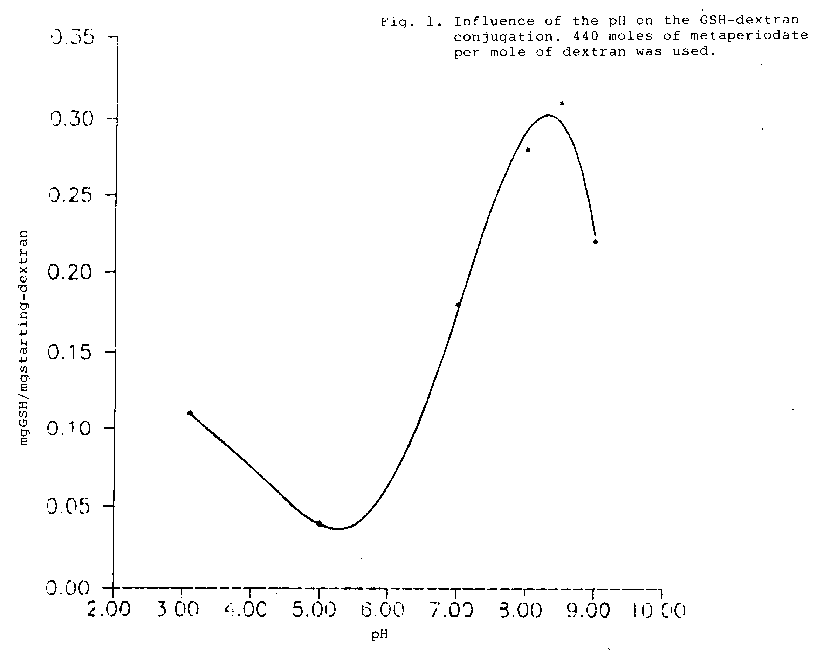

Thus, dextran was oxidized with sodium metaperiodate and the aldehydes formed were coupled to GSH under different conditions. The efficiency of th4e GSH-dextran conjugation was highly dependent on the pH (Fig. 1). Although the aldehyde groups in the oxidized dextran are more reactive towards nucleophilic agents when they are protonated, the formation of the Schiff's base depends on the presence in GSH of unprotonated amino groups. Thus, the highest extent of conjugation was achieved at pH 8.0-8.5 while in the pH range 4.0-6.5 the coupling efficiency was very low.

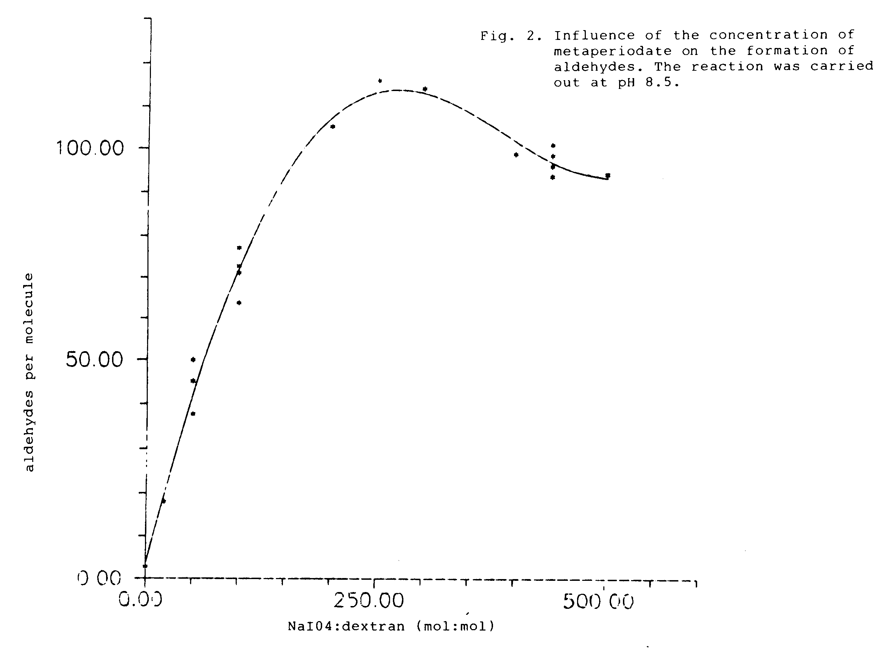

The GSH coupling capacity of the oxidized dextran depends on the number of aldehyde groups generated by an overoxidation gives rise to fragmentation of the starting polysaccharide. Therefore, the conditions of the oxidation of dextran have been carefully studied. The number of aldehyde groups formed per molecule of dextran as a function of the metaperiodate:dextran molar ratio employed is shown in Fig. 2. A maximum of 115-120 aldehyde groups was observed when using 250-300 moles of oxidant per mole of dextran. The apparent decrease of the number of groups observed at higher molar ratios could be the result of the lost upon dialysis of smal fragments of the polysaccharide, giving rise to a decreasing of the actual dextran concentration.

In accordance with the invention, the new dextran-glutathione complexes are characterised by a glutathione percentage of maximum 30%, and also by a molecular weight in the range of 14.00 to about 40.000.

In accordance with the invention, the method of preparing soluble dextran-glutathione complexes is characterized in that it comprises subjecting a soluble dextran to oxidation, then to reaction with glutathione to give the corresponding Schiff's base and finally to chemical reduction.

In accordance with the invention, the dextran is oxidized by addition of solid sodium metaperiodate and then incubation in the dark with end-over-rotation.

The amount of added sodium metaperiodate used in the oxidation stage is such as to reach a final molar ratio of 50-100 moles of oxidant per mol of dextran.

In accordance with the invention, the oxidized dextran and glutathione are reacted in phosphate buffer at pH 7,4 and the pH adjusted to the desired value with 10 N sodium hydroxide.

In accordance with the invention, the conjugation of oxidized dextran with glutathione is carried out at pH 8.0-8.5, the final chemical reduction being by incubation with sodium borohydride.

Soluble dextran-glutathione complexes, for preparing a suitable medicament for the treatment of hetatopathies, likewise comprise an object of the invention.

Several GSH conjugates have been prepared from dextrans showing different degrees of oxidation. The final product was a water soluble white or pale yellow powder with a GSH content closely related to the oxidation grade of the starting dextran (Table 1). The highest GSH coupling, 30% w/w, was obtained at the highest oxidant:dextran ratio studied (440:1 metaperiodate:dextran).

**Table 1**

| Characterization of the different dextran-GSH conjugates. | | | |
|---|---|---|---|
| oxidant:dextran ratio | & GSH (w/w) | sedimentation coefficient | MW |
| - | - | 2.4 S | 39100 |
| 1:50 | 5.9 | 2.2 S | 34000 |
| 1:100 | 13.9 | 1.9 S | 27540 |
| 1:250 | 23.6 | N.S^{a} | 14000-20000^{b} |
| 1:440 | 30.2 | N.S | 14000-20000 |

| | | | |
|---|---|---|---|
| ^{a} Not sedimented at 60000 rev/min. | | | |
| ^{b} Molecular weight between 14000, cut-off of the dialysis tubing, and 20000, minimun average molecular weight not sedimenting at 60000 rev/min in the ultracentrifuge. | | | |

These dextran GSH conjugates were tested as potential intrahepatic glutathione delivery systems. Their biological activity was evaluated by measuring the glutathione levels in isolated hepatoma cells after incubation with the different dextran-GSH conjugates either in the absence or in the presence of an inhibitor of cellular GSH synthesis (Table 2). All the conjugates displayed a protective effect on GSH depletion induced by administration of buthionine-SR-sulfoximide although an apparent decrease in activity was observed for the compounds with the lowest molecular weight.

**Tabla 2**

| Glutathione levels in H35 hepatoma cells after BSO and dextran-GSH treatment | |
|---|---|
| Addition | µg GSH/mg protein |
| Control | 22.64 ± 0.74 (4)^{a} |
| BSO | 5.27 ± 1.17 (4) |
| D1 (1:50) | 32.30 ± 0.0 (2) |
| BSO + D1 | 35.33 ± 3.1 (4) |
| D2 (1:100) | 34.80 ± 6.3 (4) |
| BSO + D2 | 28.70 ± 6.46 (3) |
| D3 (1:250) | 29.60 ± 2.5 (3) |
| BSO + D3 | 24.45 ± 2.8 (2) |
| D4 (1:440) | 37.90 ± 3.4 (4) |
| BSO + D4 | 17.30 ± 0.8 (4) |

| | |
|---|---|
| ^{a} Number of determinations. | |

### EXAMPLES

### 1. Preparation of soluble dextran-glutathione complexes.

### 1.1. Sodium metaperiodate oxidation of dextran.

A 50 mg/ml dextran solution (Pharmacia T40, M_{w} 39100 Mₙ 24500) in distilled water was oxidized by addition of solid sodium metaperiodate (20 to 500 moles per mole of dextran) and incubation in the dark with end-over-end rotation. After 1 h at rom temperature the solution was dialyzed entensively against distilled water. The number of aldehyde groups generated was determined directly in this solution by the dinitrosalicylic acid assay [P. Bernfeld, 1955 in Methods in Enzymology (S.P. Colowick, and N.O. Kaplan eds.) Academic Press, New York, 1, 149].

### 1.2. Coupling of GSH to oxidized dextran

10 ml of the oxidized dextran solution was mixed with 1 ml 0.1 M sodium phosphate buffer, pH 7.4, and 1 g GSH. The pH was adjusted to the desired value with 10 N NaOH and then 0.1 g sodium borohydride was added. After 16 h at room temperature another 40 mg NaBH₄ was added and the mixture was incubated for an additional 4 h period. Unconjugated GSH was removed by dialysis against distilled water and finally the dextran GSH conjugate was freeze-dried.

The GSH content of the product was determined by measuring sulfhydryl groups by the method fo Ellman (G.L. Ellman, Arch. Biochem. Biophys. 1959, 82, 70), using GSH as standard. The molecular mass of the conjugate was estimated by analytical ultracentrifugation. The experiments were carried out at 60000 rev/min and 20ºC, in a Beckman model analytical ultracentrifuge.

### 2. Determination of biological activity

### 2.1 Cell culture

H35 hepatoma cells were grown to confluence in DMEM with 5% fetal calf serum and 5% calf serum in 3.5 cm plates. After confluence, the medium was discarded and fresh medium containing different compounds was added:
- Control.- 1 ml of medium previously described.
- BSO.- 1 ml de medium + 10 mM buthionine-SR-sulfoximine.
- Conjugate.- 1 ml of medium + 10 mM dextran-GSH-conjugate (GSH concentration).
- BSO + conjugate.- 1 ml of medium + 10 mM BSO + 10 mM dextran-GSH conjugate (GSH concentration).

Different amounts of conjugate were added to obtain a final GSH concentration of 10 mM. After 4 hours of incubation, media were discarded and cells were washed twice with 1 ml of Hanks. Then the cells were homogenized in 750 µl of 0.1 M sodium phosphate, 0.005 mM EDTA, pH 8, and the proteins were precipitated by the addition of 200 µl of 25% phosphoric acid. Samples were centrifuged at 100000 x g, 4ºC for 30 min and supernatants were collected to determine GSH content.

### 2.2. Measurement of GSH levels

GSH levels were determined by the method described by Hissin et al with some modifications. The reactions mixtures contained 1.8 ml of phosphate-EDTA buffer, 100 µl of the supernatant obtained as described above and 100 µl of 0-ptaldialdehyde, (1 mg/ml in methanol). After incubation for 15 min at room temperature, fluorescence was determined at 420 nm with an excitation wavelength of 350 nm.

## Claims (Claims for the following Contracting State(s): DE, GB, AT, NL, BE, LU, CH/LI, DK, FR, IT, SE)

1. New soluble dextran-glutathione complexed, characterized by a glutathione percentage of maximum 30% and also by a molecular weight in the range of 14.000 to about 40.000.

2. A method of preparing soluble dextran-glutathione complexes as potential intrahepatic delivery systems comprising subjecting a soluble dextran to oxidation then to reaction with glutathione to give the corresponding Schiff's bse and finally to chemical reduction.

3. A method as claimed in claim 1 in which the dextran is oxidized by addition of solid sodium metaperiodate and then incubation in the dark with end-over-end rotation.

4. A method as claimed in claim 1 in which the amount of added sodium metaperiodate used in the oxidation step is such as to reach a final molar ratio of 50-100 moles of oxidant per mol of dextran.

5. A method as claimed in claim 1 in which the oxidized dextran and glutathione are reacted in phosphate buffer pH 7.4 and the pH adjusted to the desired value with 10 N sodium hydroxide.

6. A method as claimed in claim 1 in which the conjugation of oxidized dextran with glutathione is carried out at pH 8.0-8.5.

7. A method as claimed in claim 1 in which the final chemical reduction is carried out by incubation with sodium borohydride.

8. Soluble dextran-glutathione complexed for preparing a suitable medicament for treating hepatopathies.

## Claims (Claims for the following Contracting State(s): ES, GR)

1. A method of preparing soluble dextran-glutathione complexes as potential intrahepatic delivery systems comprising subjecting a soluble dextran to oxidation then to reaction with glutathione to give the corresponding Schiff's bse and finally to chemical reduction.

2. A method as claimed in claim 1 in which the dextran is oxidized by addition of solid sodium metaperiodate and then incubation in the dark with end-over-end rotation.

3. A method as claimed in claim 1 in which the amount of added sodium metaperiodate used in the oxidation step is such as to reach a final molar ratio of 50-100 moles of oxidant per mol of dextran.

4. A method as claimed in claim 1 in which the oxidized dextran and glutathione are reacted in phosphate buffer pH 7.4 and the pH adjusted to the desired value with 10 N sodium hydroxide.

5. A method as claimed in claim 1 in which the conjugation of oxidized dextran with glutathione is carried out at pH 8.0-8.5.

6. A method as claimed in claim 1 in which the final chemical reduction is carried out by incubation with sodium borohydride.
